# EUROPÄISCHE PATENTSCHRIFT

(11) **EP 0 480 956 B1**
(45) Veröffentlichungstag und Bekanntmachung des Hinweises auf die Patenterteilung: **10.08.1994**
(21) Anmeldenummer: 90909700.8
(22) Anmeldetag: 02.07.1990
(51) Int. Cl.: C07D 251/28

(54) **VERFAHREN ZUR HERSTELLUNG VON 2,4,6-TRIFLUOR-1,3,5-TRIAZIN**
METHOD FOR PREPARING 2,4,6-TRIFLUORO-1,3,5-TRIAZINE
PROCEDE DE PRODUCTION DE 2,4,6-TRIFLUORO-1,3,5-TRIAZINE

(30) Priorität: 04.07.1989 DE 3921918
(43) Veröffentlichungstag der Anmeldung: 22.04.1992
(73) Patentinhaber: HOECHST AKTIENGESELLSCHAFT, 65926 Frankfurt am Main (DE)
(72) Erfinder: GRÖTSCH, Georg, D-6238 Hofheim am Taunus (DE)
(86) Internationale Anmeldenummer: EP9001055
(87) Internationale Veröffentlichungsnummer: WO9100279

(56) Entgegenhaltungen:
- EP-A- 0 035 704
- The Journal of Organic Chemistry, Vol. 25, 1960, Seiten 2016-2019, Columbus, Ohio, US; C.W. Tullock et al.: "Synthesis of fluorides by metathesis with sodium floride"

## Beschreibung

Die Erfindung betrifft ein verbessertes Verfahren zur Herstellung von 2,4,6-Trifluor-1,3,5-triazin (Cyanurfluorid) in hoher Reinheit und hoher Ausbeute durch Umsetzung von Cyanurchlorid mit einem Alkalimetallfluorid in einem dipolar aprotischen Lösungsmittel bei relativ niederen Temperaturen.

Cyanurfluorid ist eine wertvolle Ausgangsverbindung für Agrochemikalien, Pharmazeutika, Farbstoffe, Photochemikalien und optische Aufheller.

Es ist bekannt, daß man Cyanurfluorid durch Fluorierung von Cyanurchlorid mit Natriumfluorid in einem dipolar aprotischen Lösungsmittel herstellen kann (Tullock, Coffman, J. Org. Chem. 25, 2016 (1960)). Hierbei wird zu einer Suspension von Natriumfluorid in Sulfolan Cyanurchlorid gegeben und die Reaktionsmischung von 45°C auf 248°C erhitzt. Das gebildete Cyanurfluorid destilliert aus der Reaktionsmischung ab. Die Ausbeute beträgt 74 % d.Th.. Nach der DE-OS 3 727 973 wird Cyanurfluorid nach der gleichen Methode gewonnen. Bereits als Mindesttemperatur der Umsetzung werden hier 75°C genannt. Nach Beispiel A und Zeilen 11 bis 15, Spalte 2 (Beschreibung) muß die Reaktionsmischung auf schließlich 220°C erhitzt werden, um Cyanurfluorid annähernd quantitativ, d.h. in 87 % Ausbeute zu erhalten. Als Nebenprodukte werden u.a. partiell fluorierte Substanzen, wie 6-Chlor-2,4-difluortriazin genannt, was vordergründig auf eine unvollkommene Umsetzung schließen läßt.

In EP-0 035 704 wird ein leicht verändertes Verfahren beschrieben. Cyanurchlorid oder gemischtchloriertefluorierte 1,3,5-Triazine werden, gegebenenfalls in Form einer Schmelze oder gelöst in einem dipolar aprotischen Lösungsmittel, einer Suspension von Natriumfluorid in einem dipolar aprotischen Lösungsmittel, insbesondere Sulfolan, zudosiert, die auf 120°C bis 220°C, insbesondere 140 bis 160°C erwärmt ist. Cyanurfluorid wird aus der Reaktionsmischung durch Destillation erhalten, wobei selbst in Gegenwart eines Zwischensieders im Vakuum gearbeitet werden muß, um das gebildete Cyanurfluorid vollständig abtrennen zu können.

Bei diesen bekannten Verfahren wird die Reaktionsmischung über einen größeren Teil der Reaktionszeit (Tullock, Coffman) über etwa 120°C bzw. über die gesamte Reaktionszeit (EP-O 035 704) bei Temperaturen über 120°C, vorzugsweise bei 140°-160°C, gehalten. Selbst unter Zusatz von Phasentransferkatalysatoren, wie z.B. 18-Krone-6 (CS 247 969, JP 61 047 465) sind Temperaturen von ca. 140°-400°C erforderlich, um in einem dipolar aprotischen Lösungsmittel aus Cyanurchlorid mit Alkalimetallfluoriden Cyanurfluorid zu erhalten.

Diese Verfahren bestätigen damit das bisher herrschende Vorurteil, daß die Herstellung von Cyanurfluorid (2,4,6-trifluor-1,3,5-triazin) aus Cyanurchlorid und Kaliumfluorid sehr hohe Reaktionstemperaturen erfordert (HOUBEN-WEYL, Band V/3, 1962).

Demgegenüber wurde nun überraschenderweise gefunden, daß man 2,4,6-Trifluor-1,3,5-triazin (Cyanurfluorid) in hoher Reinheit und praktisch quantitativer Ausbeute bei relativ niedrigen Temperaturen vorteilhaft herstellen kann, indem man 2,4,6-Trichlor-1,3,5-triazin (Cyanurchlorid) bzw. gemischtchlorierte-fluorierte 1,3,5-Triazine mit der mindestens äquivalenten Menge Natrium-, Kalium- oder Cäsiumfluorid oder eines beliebigen Gemisches dieser Alkalimetallfluoride in einem dipolar aprotischen Lösungsmittel bei einer Temperatur von etwa 30°C bis etwa 110°C, vorzugsweise etwa 50°C bis etwa 105°C, insbesondere etwa 70°C bis etwa 100°C, umsetzt.

Im einzelnen kann so verfahren werden, daß man Cyanurchlorid und/oder gemischtchlorierte-fluorierte 1,3,5-Triazine, das Alkalimetallfluorid bzw. Mischungen der Alkalimetallfluoride und das dipolar aprotische Lösungsmittel mischt und sich auf die gewünschte Reaktionstemperatur erwärmen läßt (Nutzung der bei der Umsetzung freiwerdenden Reaktionswärme) oder auf die gewünschte Reaktionstemperatur erwärmt.

Es kann jedoch auch so verfahren werden, daß das Cyanurchlorid als Feststoff, als Schmelze oder gelöst oder suspendiert in einem inerten Lösungsmittel der Suspension des Alkalimetallfluorids oder des Gemisches der Alkalimetallfluoride in einem dipolar aprotischen Lösungsmittel zudosiert wird.

Eine weitere Ausführungsform des erfindungsgemäßen Verfahrens besteht darin, daß das Alkalimetallfluorid bzw. das Gemisch aus den Alkalimetallfluoriden als Feststoff oder als Suspension in einem inerten Lösungsmittel der Mischung aus Cyanurchlorid und/oder gemischtchloriertenfluorierten 1,3,5-Triazinen und dem dipolar-aprotischen Lösungsmittel zudosiert wird.

Schließlich kann auch so gearbeitet werden, daß das Cyanurchlorid, gemischt mit oder gelöst in gemischtchlorierten-fluorierten 1,3,5-Triazinen, gegebenenfalls unter Zusatz eines inerten Lösungsmittels, der Suspension des Alkalimetallfluorides oder des Gemisches aus Alkalimetallfluoriden in einem dipolar aprotischen Lösungsmittel zudosiert wird.

Als dipolar aprotisches Lösungsmittel für das erfindungsgemäße Verfahren eignet sich jedes dipolar aprotische Lösungsmittel, das bekanntermaßen in Chlor/Fluor-Austausch Reaktionen eingesetzt werden kann und gegenüber den halogenierten Triazinen inert ist. Besonders geeignet ist das Sulfolan.

Es ist zweckmäßig, das Alkalimetallfluorid in einem etwa 5 bis etwa 50 %igen, vorzugsweise etwa 10 bis etwa 20%igen molaren Überschuß gegenüber der benötigten äquivalenten Menge, bezogen auf das Cyanurchlorid, einzusetzen. Man kann zwar auch einen über 50 % hinausgehenden molaren Überschuß anwenden, doch ist hierbei praktisch kein Vorteil mehr verbunden und die Wirtschaftlichkeit des Verfahrens leidet.

Das Verfahren kann sowohl bei Normaldruck als auch bei Unterdruck oder Überdruck durchgeführt werden.

Sofern bei den vorstehend beschriebenen Ausführungsformen des Verfahrens von "inerten Lösungsmitteln" die Rede ist, so handelt es sich hierbei um beliebige, gegenüber den Ausgangs- und Endverbindungen des Verfahrens unter den Reaktionsbedingungen inerten Lösungsmitteln, wie beispielsweise Chlorbenzol, Dichlorbenzol, Toluol, Xylol oder Tetrachlorethylen oder um das als Reaktionsmedium eingesetzte dipolar aprotische Lösungsmittel oder um ein anderes dipolar aprotisches Lösungsmittel, das ebenfalls gegenüber Ausgangs- und Endverbindungen unter den Reaktionsbedingungen inert ist.

Der besondere Vorteil des erfindungsgemäßen Verfahrens liegt vor allem darin, daß bisher die Umsetzung in einem Temperaturbereich durchgeführt wurde, in dem bereits merkliche Rückreaktion des gebildeten Cyanurfluorids stattfindet. So erhält man in Sulfolan Chlordifluortriazin bei 140°C in 5 h in 4 %iger Ausbeute aus Cyanurfluorid in Gegenwart eines 86 %igen KCl/KF-Gemisches. Dies ist ein Salzgemisch, wie es typischerweise nach Abschluß der Fluorierung von Cyanurchlorid mit Kaliumfluorid bei Einsatz 20 %igen Überschusses vorliegt. Bei 190°C erfolgt unter sonst gleichen Bedingungen raschere Umwandlung von Cyanurfluorid durch Fluor/Chlor-Austausch. Nach ca. 10 h sind bereits 25 % des Cyanurfluorids zu Chlordifluor- und Dichlorfluortriazin umgesetzt (vgl. Beispiele 1 und 2). Demgegenüber tritt dieser Nachteil - Rückreaktion des gebildeten Cyanurfluorids - nicht auf, wenn bei Temperaturen bis etwa 110°C gearbeitet wird (vgl. Beispiel 3). Es wird somit schnell und quantitativ Cyanurfluorid gebildet. Ein Gleichgewicht zwischen gemischtchlorierten-fluorierten Triazinen und Cyanurfluorid stellt sich nicht ein.

Zudem ist gegenüber den bisherigen Verfahren zur Herstellung von Cyanurfluorid aus Cyanurchlorid oder gemischtchlorierten-fluorierten 1,3,5-Triazinen mit Alkalimetallfluoriden bei hohen Temperaturen eine deutliche Energieeinsparung und damit eine wirtschaftlichere Synthese von Cyanurfluorid möglich.

Die Isolierung des Cyanurfluorids erfolgt auf an sich übliche Weise durch schonende Destillation entweder in Gegenwart des Alkalimetallchlorids oder nach Abtrennen des angefallenen Salzes, wobei gegebenenfalls Vakuum angewendet wird.

Zur Gewinnung von Cyanurfluorid hoher Reinheit ist keine aufwendige Abtrennung von gemischtchlorierten-fluorierten 1,3,5-Triazinen nötig, da diese nach Abschluß der Umsetzung nicht mehr in der Reaktionsmischung enthalten sind.

Das erfindungsgemäße Verfahren wird durch die nachstehenden Beispiele näher erläutert, ohne darauf beschränkt zu werden. (In den Beispielen wird 1,3,5-Triazin als "s-Triazin" bezeichnet.)

### Beispiel 1 (Vergleichsbeispiel)

Eine Suspension von 70,3 g KCl/KF-Mischung (86,5 Gew.-% KCl) in 138 g trockenem Sulfolan wird mit 30,6 g Cyanurfluorid bei Raumtemperatur versetzt und anschließend bei 140°C gerührt. Laut gaschromatographischer Analyse ("GC-Analyse") der Reaktionsmischung haben sich nach 5 Stunden 4 % des eingesetzten Cyanurfluorids in Chlordifluortriazin umgewandelt.

### Beispiel 2 (Vergleichsbeispiel)

Eine Reaktionsmischung, wie in Beispiel 1 beschrieben, wird bei 190°C gerührt. Nach 10 Stunden enthält die Reaktionsmischung laut GC-Analyse s-Triazine in folgender Verteilung: 75 % Cyanurfluorid, 6 % Chlordifluor-s-triazin, 19 % Dichlorfluor-s-triazin.

### Beispiel 3

Eine Reaktionsmischung, wie in Beispiel 1 beschrieben, wird 5 Stunden bei 100°C gerührt. Laut GC ist kein Fluor/Chlor-Austausch am Cyanurfluorid nachzuweisen. Das Cyanurfluorid kann quantitativ zurückgewonnen werden.

### Beispiel 4

46,1 g Cyanurchlorid werden bei 40°C zu einer Suspension von 52,3 g Kaliumfluorid in 175 g trockenem Sulfolan zugegeben und in 20 Minuten auf 80°C erwärmt. Nach 80 minütigem Rühren bei dieser Temperatur beträgt die Ausbeute laut GC 99,2 % der Theorie.

### Beispiel 5

67,5 g Cyanurchlorid werden bei 30°C mit einer Suspension von 104,6 g Kaliumfluorid in 300 g Sulfolan in einem wärmeisolierten Reaktionsgefäß gemischt. Innerhalb von 10 Minuten erwärmt sich die Reaktionsmischung gleichmäßig auf 97°C. GC-Kontrolle zeigt, daß sich Cyanurchlorid bereits quantitativ zu Cyanurfluorid umgesetzt hat.

### Beispiel 6

Zu einer Suspension von 384 g Kaliumfluorid in 700 g Sulfolan werden bei 100°C eine ebenfalls 100°C heiße Lösung von 369 g Cyanurchlorid in 500 g Sulfolan innerhalb von 2 Stunden zugetropft. GC-Kontrolle der Reaktionsmischung zeigt, daß sich Cyanurchlorid quantitativ zu Cyanurfluorid umgesetzt hat. Anschließend wird Cyanurfluorid bei einem Druck von 500 bis 100 mbar abdestilliert. Die Ausbeute beträgt 257 g (95 % d.Th.); Gehalt: 99,4 Flächen-% (GC).

### Beispiele 7 bis 10

Verfahrensdurchführung analog Beispiel 6. Einsatzmengen und Ausbeuten sind in nachfolgender Tabelle zusammengefaßt.

| Beispiel | NaF [g] | KF [g] | CsF [g] | Cyanurchlorid [g] | Sulfolan [g] | Cyanurfluorid [g] |
|---|---|---|---|---|---|---|
| 7 | 302 | - | - | 369 | 1000 | 251 |
| 8 | 134 | 13 | - | 184 | 550 | 122 |
| 9 | - | 191 | 19 | 184 | 700 | 126 |
| 10 | 139 | - | 14 | 184 | 550 | 125 |

### Beispiel 11

30 g einer Mischung aus Cyanurchlorid (8,6 Gew.-%) Dichlorfluor-s-triazin (9,5 Gew.-%), Chlordifluor-s-triazin (16,4 Gew.-%) und Cyanurfluorid (65,5 Gew.-%) werden zu 20 g KF in Sulfolan gegeben und bei 60° bis 70°C gerührt. Nach 40 Minuten sind laut GC alle chlorhaltigen Triazine in Cyanurfluorid umgewandelt.

### Beispiel 12

In einem 2 l-Autoklav wird eine Mischung aus 184 g Cyanurchlorid, 209 g Kaliumfluorid und 900 g Sulfolan 4 Stunden auf 100°C erhitzt. Es stellt sich ein Überdruck von ca. 0,8 bar ein. Laut GC erfolgte quantitativer Umsatz des Cyanurchlorids zu Cyanurfluorid.

### Beispiel 13

Bei 100°C werden 209 g Kaliumfluorid über 1 Stunde zu 184 g Cyanurchlorid in 800 g Sulfolan zudosiert und anschließend noch 1 Stunde bei dieser Temperatur gerührt. Laut GC ist der Umsatz des Cyanurchlorids zum Cyanurfluorid quantitativ.

### Beispiel 14

Analog Beispiel 6 werden 184 g Cyanurchlorid mit 192 g Kaliumfluorid umgesetzt. Nach Absaugen des nach Reaktionsende angefallenen KCl/KF-Gemisches und Nachwaschen mit 600 g Chlorbenzol wird Cyanurfluorid aus der Mutterlauge abdestilliert. Die Ausbeute beträgt 129 g (96 % der Theorie). Gehalt: 99,9 Fl-% (GC).

Der Siedepunkt des nach den Beispielen 6 bis 10 und 14 erhaltenen und isolierten Cyanurfluorids ist 72,5 bis 73,0°C.

### Beispiel 15 (Vergleichsbeispiel)

Eine 140°C heiße Lösung von 111 g Cyanurchlorid in 60 g Sulfolan wird in 15 Minuten zu einer 160°C heißen Suspension von 90,8 g NaF in 104 g Sulfolan zudosiert und dann 1 Stunde bei 190°C nachgerührt, wobei insgesamt bereits etwa 3/4 des Gesamtfluorids überdestillieren. Restliches Produkt wird bei 500 mbar bis 50 mbar abgetrennt. Die Ausbeute beträgt 74,2 g (92 % der Theorie). Laut GC setzt sich das Produkt wie folgt zusammen: Cyanurfluorid 97,7 %, Chlordifluortriazin 1,5 %, Dichlorfluortriazin 0,5 %, unbekannte Verbindungen 0,3 %.

## Patentansprüche

1. Verfahren zur Herstellung von 2,4,6-Trifluor-1,3,5-triazin (Cyanurfluorid) in hoher Reinheit und hoher Ausbeute bei relativ niederen Temperaturen, dadurch gekennzeichnet, daß man 2,4,6-Trichlor-1,3,5-triazin (Cyanurchlorid) bzw. gemischtchlorierte-fluorierte 1,3,5-Triazine mit der mindestens äquivalenten Menge Natrium-, Kalium- oder Cäsiumfluorid oder eines beliebigen Gemisches dieser Alkalimetallfluoride in einem dipolar aprotischen Lösungsmittel bei Temperaturen von etwa 30°C bis etwa 110°C umsetzt und das gebildete 2,4,6-Trifluor-1,3,5-triazin durch Destillation isoliert.

2. Verfahren nach Anspruch 1, dadurch gekennzeichnet, daß man bei Temperaturen von etwa 50°C bis etwa 105°C umsetzt.

3. Verfahren nach mindestens einem der Ansprüche 1 und 2, dadurch gekennzeichnet, daß man bei Temperaturen von etwa 70°C bis etwa 100°C umsetzt.

4. Verfahren nach mindestens einem der Ansprüche 1 bis 3, dadurch gekennzeichnet, daß man Cyanurchlorid und/oder gemischtchlorierte-fluorierte 1,3,5-Triazine, das Alkalimetallfluorid bzw. Mischungen der Alkalimetallfluoride und das dipolar aprotische Lösungsmittel mischt und sich auf die gewünschte Reaktionstemperatur erwärmen läßt oder auf die gewünschte Reaktionstemperatur erwärmt.

5. Verfahren nach mindestens einem der Ansprüche 1 bis 3, dadurch gekennzeichnet, daß das Cyanurchlorid als Feststoff, als Schmelze oder gelöst oder suspendiert in einem inerten Lösungsmittel der Suspension des Alkalimetallfluorids oder des Gemisches der Alkalimetallfluoride in einem dipolar aprotischen Lösungsmittel zudosiert wird.

6. Verfahren nach mindestens einem der Ansprüche 1 bis 3, dadurch gekennzeichnet, daß das Alkalimetallfluorid bzw. das Gemisch aus den Alkalimetallfluoriden als Feststoff oder als Suspension in einem inerten Lösungsmittel der Mischung aus Cyanurchlorid und/oder gemischtchlorierten-fluorierten 1,3,5-Triazinen und dem dipolar-aprotischen Lösungsmittel zudosiert wird.

7. Verfahren nach mindestens einem der Ansprüche 1 bis 3, dadurch gekennzeichnet, daß gemischtchloriertefluorierte 1,3,5-Triazine oder das Cyanurchlorid, gemischt mit oder gelöst in gemischtchlorierten-fluorierten 1,3,5-Triazinen, gegebenenfalls unter Zusatz eines inerten Lösungsmittels, der Suspension des Alkalimetallfluorides oder des Gemisches aus den Alkalimetallfluoriden in einem dipolar aprotischen Lösungsmittel zudosiert wird.

8. Verfahren nach mindestens einem der Ansprüche 1 bis 7, dadurch gekennzeichnet, daß man vor der Isolierung des 2,4,6-Trifluor-1,3,5-triazins die angefallenen Alkalimetallchloride abtrennt.

9. Verfahren nach mindestens einem der Ansprüche 1 bis 8, dadurch gekennzeichnet, daß man bei Normaldruck, Unterdruck oder Überdruck arbeitet.

## Claims

1. A process for the preparation of 2,4,6-trifluoro-1,3,5-triazine (cyanuric fluoride) in high yield and high purity at relatively low temperatures, which comprises reacting 2,4,6-trichloro-1,3,5-triazine (cyanuric chloride) or mixed chlorinated/fluorinated 1,3,5-triazines with at least the equivalent amount of sodium fluoride, potassium fluoride or cesium fluoride or any desired mixture of these alkali metal fluorides in a dipolar aprotic solvent at temperatures from about 30°C to about 110°C and isolating the 2,4,6-trifluoro-1,3,5-triazine formed by distillation.

2. The process as claimed in claim 1, wherein the reaction is carried out at temperatures from about 50°C to about 105°C.

3. The process as claimed in at least one of claims 1 and 2, wherein the reaction is carried out at temperatures of about 70°C to about 100°C.

4. The process as carried out in at least one of claims 1 to 3, wherein cyanuric chloride and/or mixed chlorinated/fluorinated 1,3,5-triazines, the alkali metal fluoride or mixtures of the alkali metal fluorides and the dipolar aprotic solvent are mixed and allowed to heat to the desired reaction temperature or are heated to the desired reaction temperature.

5. The process as claimed in at least one of claims 1 to 3, wherein the cyanuric chloride is metered into the suspension of the alkali metal fluoride or the mixture of the alkali metal fluorides in a dipolar aprotic solvent as a solid, as a melt or dissolved or suspended in an inert solvent.

6. The process as claimed in at least one of claims 1 to 3, wherein the alkali metal fluoride or the mixture of the alkali metal fluorides is metered into the mixture of cyanuric chloride and/or mixed chlorinated/fluorinated 1,3,5-triazines and the dipolar aprotic solvent as a solid or as a suspension in an inert solvent.

7. The process as claimed in at least one of claims 1 to 3, wherein mixed chlorinated/fluorinated 1,3,5-triazines or the cyanuric chloride, mixed with or dissolved in mixed chlorinated/fluorinated 1,3,5-triazines, is/are metered into the suspension of the alkali metal fluoride or the mixture of the alkali metal fluorides in a dipolar aprotic solvent, if desired with the addition of an inert solvent.

8. The process as claimed in at least one of claims 1 to 7, wherein the alkali metal chlorides produced are separated off before the isolation of the 2,4,6-trifluoro-1,3,5-triazine.

9. The process as claimed in at least one of claims 1 to 8, which process is carried out at normal pressure, reduced pressure or elevated pressure.

## Revendications

1. Procédé pour préparer la 2,4,6-trifluoro-1,3,5-triazine (fluorure de cyanuryle) avec une grande pureté et un grand rendement à des températures relativement basses, caractérisé en ce qu'on fait réagir la 2,4,6-trichloro-1,3,5-triazine (chlorure de cyanuryle), ou encore des 1,3,5-triazines chlorofluorées, avec une quantité au moins équivalente de fluorure de sodium, de potassium ou de césium ou d'un mélange quelconque de ces fluorures de métaux alcalins, dans un solvant aprotique dipolaire et à une température d'environ 30 à environ 110°C, la 2,4,6-trifluoro-1,3,5-triazine formée étant isolée par distillation.

2. Procédé selon la revendication 1, caractérisé en ce qu'on met en oeuvre la réaction à des températures d'environ 50 à environ 105°C.

3. Procédé selon au moins l'une des revendications 1 et 2, caractérisé en ce qu'on met en oeuvre la réaction à des températures d'environ 70 à environ 100°C.

4. Procédé selon au moins l'une des revendications 1 à 3, caractérisé en ce qu'on mélange le chlorure de cyanuryle et/ou les 1,3,5-triazines chlorofluorées, le fluorure de métal alcalin ou les fluorures de métaux alcalins et le solvant aprotique dipolaire, et on laisse la température s'échauffer à la température souhaitée de réaction, ou encore on chauffe à la température souhaitée de la réaction.

5. Procédé selon au moins les revendications 1 à 3, caractérisé en ce qu'on ajoute le chlorure de cyanuryle sous forme d'un solide, d'une masse fondue ou en solution ou en suspension dans un solvant inerte, à la suspension du fluorure de métal alcalin ou du mélange des fluorures de métaux alcalins, dans un solvant aprotique dipolaire.

6. Procédé selon au moins une des revendications 1 à 3, caractérisé en ce que le fluorure de métal alcalin ou le mélange des fluorures de métaux alcalins est ajouté, sous forme d'un solide ou d'une suspension dans un solvant inerte, au mélange de chlorure de cyanuryle et/ou de 1,3,5-triazines chlorofluorées et au solvant aprotique dipolaire.

7. Procédé selon au moins une des revendications 1 à 3, caractérisé en ce que les 1,3,5-triazines chlorofluorées ou encore le chlorure de cyanuryle, en mélange avec les 1,3,5-triazines chlorofluorées ou en solution dans ces derniéres, éventuellement avec addition d'un solvant inerte, sont ajoutés à la suspension du fluorure de métal alcalin ou du mélange de fluorures de métaux alcalins dans un solvant aprotique dipolaire.

8. Procédé selon au moins l'une des revendications 1 à 7, caractérisé en ce que, avant d'isoler la 2,4,6-trifluoro-1,3,5-triazine, on sépare les chlorures de métaux alcalins qui se sont formés.

9. Procédé selon au moins l'une des revendications 1 à 8, caractérisé en ce qu'on travaille sous la pression normale, en dépression ou en surpression.
